# EUROPÄISCHE PATENTANMELDUNG

(11) **EP 2 471 900 A2**
(43) Veröffentlichungstag der Anmeldung: **04.07.2012**
(21) Anmeldenummer: 11009651.8
(22) Anmeldetag: 02.12.2003
(51) Int. Cl.: C12M 3/00, C12M 1/26, G01N 1/28, A61B 10/00, B01L 3/02

(54) **Verfahren und Vorrichtungen zum Transfer biologischer Zellen zwischen einem Träger und einer Sonde**

(30) Priorität: 21.02.2003 DE 10307487
(62) Teilanmeldung aus: 03782284.8
(71) Anmelder: Fraunhofer-Gesellschaft zur Förderung der angewandten Forschung e.V., 80686 München (DE)
(72) Erfinder: Fuhr, Günter R., 13187 Berlin (DE); Zimmermann, Heiko, 66386 St. Ingbert (DE); Thielecke, Hagen, 15366 Neuenhagen bei Berlin (DE)
(74) Vertreter: Katzameyer, Michael

(57) **Zusammenfassung**

Es wird ein Zelltransferverfahren beschrieben, bei dem ein Transfer von mindestens einer, eine natürliche Zellbewegung ausführenden biologischen Zelle (21) zwischen einem Träger (80) und einer Sonde (10) vorgesehen ist und wobei eine Positionierung der Sonde (10) und des Trägers (80) relativ zueinander in eine Transferanordnung vorgesehen ist, bei der der Transfer der Zelle (21) aufgrund der natürlichen Zellbewegung erfolgt. Es werden auch Sonden und Zellmanipulatoren zum Transfer von biologischen Zellen beschrieben.

## Beschreibung

Die Erfindung betrifft Verfahren zur Bewegung biologischer Zellen, insbesondere zum Transfer von mindestens einer, eine natürliche Zellbewegung ausführenden biologischen Zelle zwischen einem Träger und einer Sonde, wie z. B. Zelltransferverfahren zur Entnahme oder zum Einbringen von biologischen Zellen oder Zellgruppen in oder aus Zellmaterial, Manipulations- und/oder Untersuchungseinrichtungen, die zur Durchführung derartiger Verfahren eingerichtet sind, insbesondere Sonden zum Transfer von mindestens einer biologischen Zelle von oder zu einem Träger, Zellmanipulatoren, die mindestens eine derartige Manipulations- und/oder Untersuchungseinrichtung aufweisen, und Anwendungen der genannten Verfahren.

In der Medizin, Biotechnologie und Biochemie gibt es zahlreiche Verfahren, bei denen biologische Zellen untersucht oder bearbeitet oder zur Untersuchung oder Bearbeitung (Veränderung) biologischen Materials verwendet werden. Beispielsweise werden zur medizinischen Zelltherapie einem tierischen oder menschlichen Probanden Zellen entnommen, außerhalb des Probandenkörpers behandelt, gesammelt, sortiert und/oder kultiviert, um anschließend bestimmte Zellen oder Zellgruppen in den Probanden zurückzuführen. Besondere Vorteile erwartet man von der medizinischen Zelltherapie mit Stammzellen, da diese die Fähigkeit zur Differenzierung zu nahezu allen Zelltypen des Körpers besitzen und daher Kandidaten für individuelle Zelltherapien und für die *in vitro* Regenerierung von Gewebe darstellen. Heute geht man davon aus, dass adulte oder embryonale Stammzellen unter geeigneten Bedingungen zu nahezu allen Zellleistungen des Körpers und damit auch zur Bildung oder Regeneration verschiedener Gewebe geeignet sind. Es besteht daher ein starkes Interesse an einer sicheren und reproduzierbaren Handhabung biologischer Zellen.

Wesentliche Aufgaben bei der Untersuchung oder Manipulation biologischer Zellen, insbesondere in Zusammenhang mit der medizinischen Zelltherapie und der Gewebebearbeitung (Tissue Engineering) bestehen darin, dass an vorbestimmten Orten bspw. im Gewebe oder in einem Zellverband mit einer Genauigkeit im µm-Bereich einzelne, vorher auswählbare Zellen oder Zellgruppen eingefügt oder entnommen oder definierte Messungen durchgeführt werden können. Bisher werden z. B. Stammzellen mit einer Injektionsspritze in ein Zielgewebe überführt. Diese Aufgaben müssen ohne eine Beeinträchtigung oder Schädigung der Zellen oder des Gewebes mit hoher Reproduzierbarkeit, Steuerbarkeit und Genauigkeit lösbar sein. Diese Anforderungen werden jedoch bisher nicht befriedigend erfüllt.

Aus der Praxis ist bekannt, dass bei Tierversuchen trotz gleichartig durchgeführter Verfahren, z. B. zur Injektion von Zellen in erkranktes Gewebe widersprüchliche Ergebnisse erzielt wurden. Es wurde festgestellt, dass der positive Verlauf einer Geweberegeneration empfindlich von den Verfahrensbedingungen, insbesondere von der Art der Injektion, der Zahl der eingebrachten Zellen oder Substanzen und der verwendeten Injektionswerkzeuge abhängt. Bei zahlreichen Experimenten trat nicht die gewünschte Regeneration oder Neubildung eines Zell- oder Gewebetyps ein, sondern bspw. eine Induktion von Tumoren. Es wird davon ausgegangen, dass die Induktion von Tumoren als unkontrollierte Zellvermehrung von Stammzellen durch physikalische, chemische oder mechanische Fremdeinflüsse am Injektionsort befördert wird. Diese Einflüsse können mit den herkömmlichen Injektionstechniken nicht reproduzierbar eingestellt oder wenigstens erfasst werden.

Eine stressfreie und selektive Entnahme von adhärent wachsenden Zellen aus *in vitro* Kulturen bereitet die folgenden Probleme. Erstens ist ein mechanisches Abschaben der gewünschten Zellen möglich. Dabei tritt jedoch das o.g. Schädigungsrisiko auf. Alternativ kann eine Ablösung durch Trypsinierung vorgesehen sein, die allerdings einen starken, unphysiologischen biochemischen Eingriff in die Zellkultur mit der Folge darstellt, dass die Adhäsionsproteine der abgelösten Zellen neu exprimiert werden müssen. Außerdem stellt die Trypsinierung ein Problem für die Selektivität der Zellentnahme dar. Ausgewählte Zellen können nach der Trypsinierung nur schwer wiedergefunden und einzeln aus der Kultur entnommen werden.

Die genannten Probleme bei der Zelltherapie und die bisher zum Teil unbefriedigenden Ergebnisse beim Tissue Engineering stellen gegenwärtig die wichtigsten Beschränkungen und Verzögerungen vor einer breiten Anwendung dieser Verfahren in der Biotechnologie und Medizin dar.

Die Aufgabe der Erfindung ist es, verbesserte Verfahren zur Bewegung biologischer Zellen relativ zu einem Fremdkörper, wie zum Beispiel einem Träger und einer Sonde bereitzustellen, mit denen die Nachteile der herkömmlichen Verfahren überwunden werden und die insbesondere eine schonende Zellübertragung zwischen dem Träger und der Sonde ermöglichen, wobei mechanische oder biochemische Einflüsse auf die übertragene Zelle oder auf Zellen, die ggf. auf dem Träger oder der Sonde vorhanden sind, minimiert werden sollen. Es sollen insbesondere unphysiologische Eingriffe, wie eine Trypsinierung in das Zellmaterial vermieden werden. Die Aufgabe der Erfindung ist es auch, verbesserte Manipulations- und/oder Untersuchungseinrichtungen (Sonden) zur Durchführung derartiger Verfahren und Zellmanipulatoren bereitzustellen, die mit mindestens einer derartigen Sonde ausgestattet sind, mit denen die Nachteile herkömmlicher Injektions- oder Biopsievorrichtungen überwunden werden können. Die Aufgabe der Erfindung ist es auch, neue Anwendungen von Sonden zum Transfer von Zellen anzugeben.

Diese Aufgaben werden mit Verfahren, Sonden und Zellmanipulatoren mit den Merkmalen gemäß den Patentansprüchen 1, 20 oder 36 gelöst. Vorteilhafte Ausführungsformen und Anwendungen der Erfindung ergeben sich aus den abhängigen Ansprüchen.

Verfahrensbezogen basiert die Erfindung auf der allgemeinen technischen Lehre, ein Zelltransferverfahren, bei dem mindestens eine biologische Zelle, die eine natürliche Eigenbeweglichkeit besitzt und beim Kontakt mit festen Oberflächen auf diesen eine Zellbewegung ausführt, zwischen einem Träger und einer Sonde übertragen wird, dahingehend weiterzuentwickeln, dass die Sonde und der Träger zueinander benachbart oder aneinandergrenzend so angeordnet werden, dass die mindestens eine biologische Zelle sich durch die natürliche Zellbewegung vom Träger auf die Sonde (oder umgekehrt von der Sonde auf den Träger) bewegt. Die gegenseitige Ausrichtung des Trägers und der Sonde wird als Transferanordnung bezeichnet. Die Erfinder haben festgestellt, dass der Transfer aufgrund der hohen Zellbeweglichkeit überraschenderweise für die Praxis genügend schnell (z. B. innerhalb von 1 Stunde oder weniger) erfolgen kann.

Unter natürlicher Zellbewegung (englisch: cell locomotion) wird hier allgemein die Ortsänderung einer kompletten Zelle auf einer festen Oberfläche oder in Zellmaterial durch eine Umordnung von Adhäsionskontakten von Zellorganen (Membranorgane, zum Beispiel Membranausstülpungen) verstanden, wie sie beispielsweise von M. Abercrombie et al. in der Publikation "The Locomotion Of Fibroblasts In Culture" ("Experimental Cell Research", Bd. 67, 1971, S. 359-367) und von L. P. Cramer in der Publikation "Organization and polarity of actin filament networks in cells: implications for the mechanism of myosinbased cell motility" ("Biochem. Soc. Symp." Bd. 65, 1999, S. 173-205) beschrieben wird. Erfindungsgemäß erfolgt die Zellbewegung direkt von der Oberfläche der Sonde auf die Oberfläche des Trägers (oder umgekehrt), ohne dass sich die Zelle gleichzeitig von beiden Oberflächen löst.

Unter Zellmaterial wird hier allgemein eine Vielzahl von Zellen verstanden, die über Adhäsionskontakte (makromolekulare chemische Verbindungen, keine van der Waals-Bindungen) mit ihrer Umgebung in Verbindung stehen. Das Zellmaterial ist bspw. ein Verbund oder Zusammenschluss von Einzelzellen, eine Zellkultur, ein Gewebe (Verbund gleichartig differenzierter Zellen) oder ein Organ. Das Zellmaterial, insbesondere der nichtflüssige Verbund von Einzelzellen kann zusätzliche synthetische Komponenten, zum Beispiel ein synthetisches Matrixmaterial enthalten. Vorteilhafterweise ergibt sich damit ein breiter Anwendungsbereich der Erfindung. Die transferierten Zellen umfassen vorzugsweise tierische oder humane Zellen, wie zum Beispiel Fibroblasten, Makrophagen, Lymphozyten, Tumorzellen oder Nervenzellen.

Besondere Vorteile können sich ergeben, wenn das erfindungsgemäße Verfahren an Zellmaterial ausgeführt wird, das sich außerhalb eines tierischen oder menschlichen Organismus befindet. Das Zellmaterial kann unter geeigneten Kultivierungsbedingungen auf dem Träger angeordnet sein. Die Positionierung des Zellmaterials und der Sonde kann vereinfacht und mit erhöhter Präzision erfolgen.

Alternativ kann sich das Zellmaterial im Verbund in einem lebenden Organismus befinden. Die Sonde kann bspw. als Untersuchungssonde, Biopsiewerkzeug oder Injektionswerkzeug auf Gewebe aufgesetzt oder in Gewebe eingeführt werden. Die Einführung erfolgt wegen der geringen Vortriebsgeschwindigkeit bei der Positionierung oder einer laufenden Relativbewegung (siehe unten) in einem Zustand, in dem das betroffene Gewebe fixiert, z. B. mit dem umliegenden Teil des Organismus ortsfest auf einem Träger gehaltert ist. Die Verwendung eines Narkosemittels ist für die Ruhestellung bevorzugt, mit Blick auf die Verletzungsfreiheit des Verfahrens jedoch nicht zwingend erforderlich.

Die Sonde und der Träger sind allgemein separate Fremdkörper oder Objekte aus einem in Bezug auf die Zelle oder das Zellmaterial abgrenzbaren Material mit einer festen Oberfläche. Die Sonde kann insbesondere eine Manipulations- und/oder Untersuchungseinrichtung wie eine Injektionskapillare umfassen. Der Träger kann insbesondere einen an sich bekannten Kulturträger mit einzelnen Zellen oder einer Zellkultur, wie z. B. eine Glas- oder Kunststoffschale umfassen.

Die Erfinder haben festgestellt, dass die natürliche Zellbewegung und Fluktuation in der makromolekularen Bindung an Oberflächen überraschenderweise dazu ausgenutzt werden kann, dass die Zelle sich von einem Festkörper (z. B. dem Träger) auf einen anderen, separaten Festkörper (z. B. die Sonde) bewegen kann. Vorteilhafterweise ist es damit möglich, allein durch die Positionierung der Sonde und des Trägers in der Transferanordnung eine oder mehrere Zellen beispielsweise vom Träger aufzunehmen, ohne dass dabei die biochemischen oder mechanischen Bedingungen für Zellen, die auf dem Träger verbleiben, oder für die bewegte Zelle nachteilig gestört werden. Einzelzellen oder Zellgruppen können verletzungsfrei mit der Sonde aufgenommen oder abgelegt werden. Die beteiligten Zellen bleiben bei der Positionierung von Sonde und Träger in ihrem physikalischen und chemischen Zustand unverändert. Der verletzungsfreie Transfer ist insbesondere dann gegeben, wenn bei der Bewegung der Zelle diese und ggf. umgebende Zellen einer Zellkultur zwar deformiert werden und ggf. ihre räumliche Lage ändern, dabei jedoch keine chemischen Signale in Form von Botenstoffen oder Substanzabsonderungen abgeben.

Die Erfindung basiert ferner auf den folgenden Überlegungen der Erfinder. Es wurde erstens erkannt, dass die bisher verschiedenartig ausfallenden Reaktionen bspw. injizierter Zellen in einem Gewebe oder einen Zellverband darin begründet liegen, dass durch die herkömmliche Einführung eines Injektionswerkzeugs Zellen im vorhandenen Zellmaterial verletzt oder zerstört und damit Wundeffekte hervorgerufen werden. Wunden repräsentieren biochemisch andere Randbedingungen, als dies im unverletzten Gewebe der Fall wäre. Bei einer Zell- oder Gewebeverwundung werden chemische Signale (Aussendung molekularer Botenstoffe) oder zellulär getragene Prozesse, wie z. B. eine Fibroblasteneinwanderung, eine Fibronektinaussonderung oder dgl. erzeugt. Die Reaktion verletzter Zellen beeinflusst die Wirkung der injizierten Zellen oder Zusatzstoffe. Beispielsweise verhalten sich Stammzellen in der Umgebung einer Zellverwundung anders als Stammzellen in einem intakten Zellmaterial. Zweitens haben die Erfinder festgestellt, dass entgegen bisherigen Vorstellungen selbst adhäsiv an ein Substrat gebundene Zellen verletzungsfrei auf ein benachbartes Substrat wandern können.

Durch die natürliche Eigenbewegung der Zelle können die o. g. Anforderungen vollständig erfüllt werden. Weder ein Zielgewebe noch die einzusetzenden individuellen Zellen werden geschädigt oder beeinträchtigt. Der physikalische, chemische und mechanische Zustand der Zellen kann vollständig charakterisiert werden. Schädliche Kontakte zwischen Zellen und Oberflächen von Fremdkörpern werden vermieden, zelluläre Signale durch unerwünschte Oberflächenberührungen werden unterbunden. Durch die verletzungsfreie Bewegung erfolgt die Zellmanipulation ideal schonend. Die Sonde kann zielgenau an einen vorbestimmten Ort im Zellmaterial auf dem Träger geführt werden.

Gemäß einer ersten bevorzugten Ausführungsform der Erfindung wird das Zelltransferverfahren bei der Entnahme mindestens einer biologischen Zelle vom Träger angewendet. Die Sonde bildet ein Biopsiewerkzeug. Vorteilhafterweise wird ein unphysiologisch schnelles, mechanisches Abreißen von Adhäsionskontakten oder deren biochemische Unterbrechung mittels Trypsinierung vermieden. Wenn auf dem Träger Zellmaterial angeordnet ist, aus dem die Zelle entnommen und auf die Sonde transferiert wird, ergibt sich der zusätzliche Vorteil, dass auch dieses Zellmaterial in Bezug auf seine biochemischen Bedingungen ungestört und unverletzt bleibt. Bei dieser Ausführungsform können sich Vorteile für die Gewinnung nicht-modifizierter, physiologischer Zellen ergeben.

Wenn gemäß einer abgewandelten Variante der Erfindung auf oder in der Sonde mindestens ein Sensor angeordnet ist, wird vorteilhafterweise ermöglicht, dass unmittelbar nach oder vor dem Transfer mindestens eine Eigenschaft der Zelle, wie z. B. das Membranpotential, die Aussonderung von Substanzen, die Impedanz der Zelle oder Fluoreszenz gemessen werden kann. Es kann sofort entschieden werden, ob die auf der Sonde befindliche Zelle vorbestimmten Auswahlkriterien genügt oder nicht. Die Untersuchung der Zelle auf der Sonde besitzt insbesondere Vorteile für die Automatisierung des erfindungsgemäßen Zelltransferverfahrens.

Allgemein ist nach dem Zelltransfer vorzugsweise vorgesehen, dass die Sonde mit der Zelle z. B. zu einem Zielsubstrat bewegt wird. Die Entfernung der Sonde mit der Zelle vom Träger besitzt den Vorteil, dass die weitere Manipulation der entnommenen Zelle unabhängig von der Handhabung des Trägers, insbesondere von der weiteren Einstellung von Kulturbedingungen am Träger durchgeführt werden kann. Die Entfernung der Sonde mit der Zelle kann insbesondere in Abhängigkeit von der Messung von Zelleigenschaften auf der Sonde vorgesehen sein.

Im Gegensatz zur o. g. ersten Ausführungsform der Erfindung kann gemäß einer alternativen Variante vorgesehen sein, dass die mindestens eine biologische Zelle von der Sonde auf den Träger transferiert wird. Vorteilhafterweise wird auch in diesem Fall aufgrund der Eigenbewegung der Zelle eine Verletzung der sich bewegenden Zelle oder von Zellmaterial auf den Träger ausgeschlossen. Wenn sich gemäß einer bevorzugten Anwendung der Erfindung auf dem Träger Zellmaterial befindet, kann die Sonde an das Zellmaterial angrenzend oder in dieses hineinragend positioniert werden, wobei eine Verletzung des Zellmaterials vermieden wird.

Diese Alternative kann besondere Vorteile für die beabsichtigten Anwendungen in der Biotechnologie und Medizin ergeben, da die Zelle bei einer Einbettung in das Zellmaterial dieses in einem physiologischen unverletzten Zustand vorfindet. Die mindestens eine Zelle wird auf oder in das unverletzte Zellmaterial eingebettet. Es können insbesondere Stammzellen in Gewebe eingepflanzt werden, um eine gewebespezifische Differenzierung der Stammzellen zu bewirken. Degenerationen oder Tumorbildungen können unterdrückt werden.

Wenn sich gemäß einer bevorzugten Ausführungsform der Erfindung die Sonde und der Träger in der Transferanordnung gegenseitig berühren, können sich Vorteile in Bezug auf die Genauigkeit der Sondenpositionierung und die Geschwindigkeit des Zelltransfers ergeben. Vorteilhafterweise kann die gegenseitige Berührung der Sonde und des Trägers auch dann vorgesehen sein, falls auf dem Träger eine Zellkultur angeordnet ist. In diesem Fall werden Zellen der Zellkultur durch die Sonde bei der Positionierung verletzungsfrei verdrängt (siehe unten). Wenn alternativ die Sonde und der Träger, auf dem sich vor dem Transfer bereits ein Zellmaterial befindet, zum Zelltransfer mit einem Abstand angeordnet werden, der im wesentlichen gleich der Ausdehnung des Zellmaterials über der Oberfläche des Trägers oder geringer als diese ist, können sich Vorteile für ein zielgenaues Aufsetzen oder Einbetten von Zellen auf oder in das Zellmaterial ergeben.

Gemäß einer weiteren, besonders bevorzugten Ausführungsform der Erfindung kann die Relativposition der Sonde und des Trägers vor und/oder während der Zellübertragung verändert werden. Wenn die Transferanordnung zwischen Träger und Sonde sich ändert, können damit vorteilhafterweise die Selektivität und Geschwindigkeit des Zelltransfers beeinflusst werden.

Die Relativgeschwindigkeit von Sonde und Träger wird vorzugsweise kleiner oder gleich der Geschwindigkeit der natürlichen Zellbewegung (1 µm/h bis 1 mm/h) gewählt.

Wenn bei der gegenseitigen Ausrichtung der Sonde und des Trägers vorgesehen ist, dass die Sonde Zellmaterial auf dem Träger zunächst nicht berührt und dann nach Auswahl einer bestimmten Zelle aus dem Zellmaterial eine zeitweilige Kontaktbildung ausschließlich der ausgewählten Zelle mit der Sonde erfolgt, kann nach der Kontaktbildung der Abstand vergrößert werden, ohne dass der Kontakt zwischen der ausgewählten Zelle und der Sonde abreißt. Die Erfinder haben festgestellt, dass die Zellorgane überraschenderweise sogar über Freiräume (z. B. 50 µm) Verbindungen aufrechterhalten und durch die natürliche Zellbewegung überwinden können. Bei dieser Variante wird nur die ausgewählte Zelle transferiert, während die anderen Zellen auf dem Träger verbleiben. Diese Ausführungsform ist umgekehrt realisierbar, um beispielsweise nur eine von mehreren Zellen auf der Sonde hin zum Träger zu transferieren.

Des Weiteren kann eine Relativbewegung von Sonde und Träger vorteilhafterweise eine Beschleunigung des Zelltransfers ermöglichen. Beispielsweise wird beim Transfer vom Träger zur Sonde diese entgegengesetzt zur Wanderungsrichtung der Zelle unter die Zelle geschoben. Umgekehrt kann beim Transfer auf den Träger die Sonde entgegengesetzt zur Zellbewegung zurückgezogen werden. Dabei kann die Sonde in das Zellmaterial auf dem Träger ragen. Die Relativbewegung erfolgt derart, dass die Sonde die Zellen oder das Zellmaterial verletzungsfrei verdrängt oder sich von der Zelle oder dem Zellmaterial verletzungsfrei löst. Die Sonde wird beispielsweise so im Zellmaterial betätigt, dass die Zellen von der Oberfläche der Sonde auseinander geschoben oder voneinander getrennt werden, so dass Raum für die Sonde geschaffen wird, wobei die Zellen bei der Verschiebung oder Trennung in ihrem physikalischen und chemischen Zustand unverändert bleiben. Eine verletzungsfreie Verdrängung von Zellen ist insbesondere dann gegeben, wenn bei der Bewegung der Sonde die Zellen in direktem Kontakt mit dem Sondenkörper oder tiefer im Zellmaterial liegende Zellen zwar deformiert werden oder ihre räumliche Lage verändern, dabei jedoch keine chemischen Signale z. B. in Form von Botenstoffen oder Substanzabsonderungen abgeben.

Dies ermöglicht die mechanische Einführung der Sonde in Zellmaterial entgegengesetzt zur Bewegungsrichtung einer ausgewählten Zelle. Die auf dem Träger verbleibenden Zellen bleiben bei der Bewegung der Sonde durch das Zellmaterial unverletzt, wenn die Vortriebsgeschwindigkeit genügend klein ist, so dass sich die Adhäsionskontakte zwischen den Zellen auf natürliche, d. h. die Zellen nicht beeinflussende oder zerstörende Weise lösen und in der sich verändernden Umgebung neu bilden lassen.

Wenn für den erfindungsgemäßen Zelltransfer eine Sonde mit einer langgestreckten Form verwendet wird, kann es vorteilhaft sein, wenn die Sonde relativ zum Träger in einer Richtung bewegt wird, die parallel zur Ausrichtung einer langgestreckten Form der Sonde verläuft. Dabei können sich Vorteile in Bezug auf die verletzungsfreie Verdrängung der Zellen ergeben. Die Verdrängung muss lediglich an der Vorderseite der Sonde erfolgen, die im Vergleich zur übrigen Oberfläche einen sehr kleinen Flächenausschnitt darstellt. Des Weiteren besitzt diese Ausführungsform den Vorteil, mit herkömmlichen Injektionstechniken unter Verwendung von Spritzen, Kanülen oder Kapillaren kompatibel zu sein. Die Sonde kann mit an sich verfügbaren Einrichtungen zur Manipulation von Zellen oder Zellsuspensionen kombiniert werden. Es wird insbesondere ermöglicht, dass mit der Sonde eine Substanz in das Zellmaterial zugeführt wird. Vorteilhafterweise können hierzu an sich bekannte Flüssigkeitsfördereinrichtungen, wie z. B. Pumpen oder dgl. verwendet werden.

Gemäß einer bevorzugten Ausführungsform der Erfindung wird die Sonde mit einer Vortriebsgeschwindigkeit bewegt, die kleiner oder gleich einer Bezugsgeschwindigkeit ist, die durch die physiologische Bindungsrate biologischer Zellen bestimmt wird (Bewegungs- oder Bindungsgeschwindigkeit der Zellen). Bei Einstellung dieser Vortriebsgeschwindigkeit kann die Sonde vorteilhafterweise verletzungsfrei durch Zellen in einem natürlich gegebenen Verbund bewegt werden. Die Vortriebsgeschwindigkeit wird an die permanent im Gewebe stattfindende Zellbewegung angepasst. Es ist bspw. bekannt, dass sich bestimmte Typen von Immunzellen (z. B. Makrophagen) durch Verdrängung vorhandener Zellen selbst durch dichtes Gewebe bewegen. Die Erfinder haben festgestellt, dass überraschenderweise diese Verdrängungsbewegung auch mit Sonden, die erheblich größer als Immunzellen sind und makroskopische Dimensionen im Sub-Millimeter- bis Zentimeterbereich besitzen, bei Einstellung der genannten Vortriebsgeschwindigkeit realisierbar ist. Während der Sondenbewegung werden laufend makromolekulare Bindungen (zum Beispiel membranständige Makromoleküle der Integrin- und Catherinfamilie) zwischen den Zellen getrennt und zum Beispiel mit der Sondenoberfläche neu geknüpft.

Die physiologische Bezugsgeschwindigkeit ist an sich bekannt (siehe z. B. G. Fuhr et al. in "Biol. Chem.", 1998, Bd. 379, S. 1161-1173) oder an tierischen oder humanen Zellen messbar. Die interessierende Bindungsrate ist bspw. durch Messung der Dynamik von Adhäsionsmustern einzelner Zellen auf künstlichen Oberflächen ableitbar.

Besondere Vorteile der Erfindung ergeben sich, wenn die Vortriebsgeschwindigkeit der Sonde in einem Geschwindigkeitsbereich von 0.1 µm/h bis 1 mm/h, vorzugsweise im Bereich von 1 µm/h bis zu 500 µm/h gewählt wird. Diesem Geschwindigkeitsbereich entsprechen die Bindungsraten des Aufbaus und des Abbaus von makromolekularen Bindungen, die typischerweise durch membranständige Makromoleküle der Integrin- und Catherinfamilie vermittelt werden. Die bevorzugten Geschwindigkeitsbereiche entsprechen den Geschwindigkeiten der Zellbewegung von insbesondere Fibroplasten, Makrophagen, Lymphozyten, Chondrozyten oder Tumorzellen. Vorteilhafterweise kann bei Einstellung einer derart geringen Vortriebsgeschwindigkeit die Position der Sonde mit einer hohen Genauigkeit von bis zu +/-1 µm eingestellt werden. Die Vortriebsgeschwindigkeiten in den genannten Bereichen entsprechen den aktiven endogenen Bewegungsgeschwindigkeiten von Zellen in und auf Gewebe. Die Bewegung der Sonde bewirkt damit einen permanenten An- und Umbau der Zellen in der unmittelbaren Umgebung der Sondenoberfläche, wobei durch die permanent wirkende Vortriebskraft eine Verdrängung der Zellen gefördert wird.

Vorteilhafterweise können je nach Anwendungsfall verschiedene Bewegungstypen der Sonde, insbesondere Bewegungen mit einem Netto-Vorschub oder einem Netto-Rückzug, diskontinuierliche oder in Teilabschnitte zerlegte Bewegungen, oszillatorische, gleichförmige oder beschleunigte Bewegungen realisiert werden.

Wenn die Positionierung und/oder Bewegung von Träger und Sonde relativ zueinander in Abhängigkeit vom Ergebnis einer Bildaufnahme- und -verarbeitung gesteuert werden, können sich Vorteile für eine gezielte Grobpositionierung und/oder Gegenbewegung der Sonde relativ zur natürlichen Zellbewegung ergeben, so dass der Zelltransfer beschleunigt werden kann. Wenn zum Beispiel durch eine mikroskopische Beobachtung zunächst die Position einer ausgewählten, zu übertragenden Zelle erfasst wird, kann mit einer schnellen Bewegung eine Startposition für die Einstellung der Transferposition angefahren werden. Eine Bewegungserfassung umfasst eine Messung der natürlichen Zellbewegung. Sie ermöglicht vorteilhafterweise die Erzeugung von Steuersignalen für eine Antriebseinrichtung des Trägers oder der Sonde. In diesem Fall kann die automatisierte Entnahme von Zellen aus einer Zellkultur oder die Ablage von Zellen, zum Beispiel mit einem Regelkreis vereinfacht werden.

Ein weiterer Vorteil der Erfindung besteht darin, dass der Zelltransfer sequenziell oder parallel mit einer Vielzahl von Zellen durchführbar ist. Es können insbesondere die folgenden Varianten einzeln oder in Kombination vorgesehen sein. Erstens kann nach einem ersten Zelltransfer von einem Träger auf die Sonde ein weiterer Zelltransfer von der Sonde auf einen weiteren Träger, wie z. B. ein Zielsubstrat oder in einer Untersuchungseinrichtung vorgesehen sein. Zweitens können mehrere Zellen aufeinanderfolgend auf eine Sonde aufwandern und gemeinsam vom Träger entfernt werden. Drittens kann die Sonde so gestaltet werden, dass mehrere Zellen gleichzeitig auf die Sonde übertragen werden. In diesem Fall können sich Vorteile in Bezug auf die Geschwindigkeit der Zellmanipulation ergeben.

Vorrichtungsbezogen wird die o. g. Aufgabe durch eine Sonde zum Zelltransfer von oder zu einem Träger gelöst, die einen relativ zum Träger positionierbaren Sondenkörper aufweist, an dessen zum Träger weisenden Ende ein Aufnahmewerkzeug vorgesehen ist, das eine Aufnahmefläche zum adhärenten Anhaften von mindestens einer biologischen Zelle aufweist. Die Sonde besitzt den Vorteil, dass zur Durchführung des erfindungsgemäßen Zelltransfers das Aufnahmewerkzeug an sich passiv gebildet ist und keine bewegten Teile, wie z. B. mikrochirurgische Elemente benötigt. Das Aufnahmewerkzeug stellt lediglich einen eigenständigen, miniaturisierbaren Zellträger dar, auf den oder von dem die mindestens eine Zelle beim Zelltransfer wandert.

Gemäß einer bevorzugten Ausführungsform der Erfindung kann der Sondenkörper eine langgestreckte, longitudinale Form wie eine Stange oder ein Stab besitzen, an dessen freien Ende das Aufnahmewerkzeug angeordnet ist, wobei der Sondenkörper mit dem entgegensetzten Ende an einer Antriebseinrichtung angebracht werden kann. Vorteilhafterweise werden mit dieser Gestaltung Sichtbehinderungen bei einer optischen Beobachtung von Zellmaterial und des Zelltransfers vermieden. Des Weiteren können sich Vorteile für eine Vortriebsbewegung der Sonde in eine Richtung parallel zur Form des Sondenkörpers ergeben.

Gemäß einer weiteren, bevorzugten Ausführungsform der Erfindung ist das Aufnahmewerkzeug als Teil des Sondenkörpers geformt. In diesem Fall ergibt sich ein besonders einfacher Aufbau der Sonde.

Wenn der Sondenkörper gemäß einer weiteren Abwandlung einen Hohlraum besitzt, der sich z. B. entlang der longitudinalen Form erstreckt, können sich zusätzliche Vorteile für die Schaffung bestimmter Umgebungsbedingungen am Aufnahmewerkzeug, wie z. B. die Bereitstellung einer Hüllflüssigkeit, oder für die Untersuchung der Zelle auf der Sonde ergeben. Zur Bildung einer Sonde mit einem Hohlraum kann zum Beispiel vorgesehen sein, dass der Sondenkörper eine entlang einer geraden Achse langgestreckte Form (Kapillare, Rohr, Hohlnadel, Schlauch) mit einem ersten Ende, an dem das Aufnahmewerkzeug vorgesehen ist, und einem zweiten Ende besitzt, das mit der Antriebseinrichtung und ggf. einer Reservoireinrichtung verbunden sein kann. Diese Ausführungsform der Erfindung kann wegen ihrer Kompatibilität mit herkömmlichen Injektionswerkzeugen Vorteile besitzen.

Bei einem Sondenkörper mit einem Innenraum kann das Aufnahmewerkzeug im Sondenkörper (Hohlkörper) angeordnet sein. Das Aufnahmewerkzeug kann im Hohlkörper verschiebbar beweglich angeordnet sein, so dass es beim Zelltransfer am freien Ende des Hohlkörpers hervorsteht und nach Aufnahme einer Zelle in den Hohlkörper zurückgezogen werden kann.

Das Aufnahmewerkzeug bildet allgemein eine Aufnahmefläche, die gemäß bevorzugten Ausführungsformen der Erfindung eine konkave oder konvexe Form besitzt. Die konkave Form bietet den Vorteil einer räumlichen Abgrenzung der Aufnahmefläche vom übrigen Sondenkörper. Damit kann eine unerwünschte Zellbewegung auf der Oberfläche des Sondenkörpers verhindert werden. Das konkave Aufnahmewerkzeug ist beispielsweise schaufel- oder löffelförmig gebildet. Die konvexe Form kann für eine verletzungsfreie Bewegung der Sonde durch Zellmaterial von Vorteil sein. Ein konvexes Aufnahmewerkzeug mit einer Kugelfläche als Aufnahme ist vorzugsweise bei einer Sonde mit dem im Hohlkörper verschiebbaren Aufnahmewerkzeug vorgesehen.

Alternativ können komplexer geformte Aufnahmewerkzeuge vorgesehen sein, falls mit dem Aufnahmewerkzeug Zusatzfunktionen beispielsweise zur Messung an der Zelle oder zur Abgrenzung der Zelle vom umgebenen Zellmaterial erfüllt werden sollen. Beispielsweise kann das Aufnahmewerkzeug ringförmig gebildet sein, wobei die Aufnahmefläche auf der Innenseite des Ringes vorgesehen ist.

Eine vorteilhafte Abwandlung ist mit einem schalen- oder kugelförmigen Aufnahmewerkzeug gegeben, dessen Innenseite die Aufnahmefläche bildet. Wenn auf der zum Träger weisenden Seite dieses Aufnahmewerkzeugs eine Öffnung vorgesehen ist, kann es mit Vorteil auf einen Träger oder das auf dem Träger befindliche Zellmaterial aufgesetzt und anschließend zurückgezogen werden, sobald sich mindestens eine Zelle auf die Innenfläche der Schalen- oder Halbkugelform bewegt hat. Durch die Ringform des Aufnahmewerkzeugs wird bei jeder Richtung der natürlichen Zellbewegung der Zelltransfer nach kurzer Zeit erreicht.

Gemäß einer weiteren Abwandlung der Erfindung kann vorgesehen sein, dass das Aufnahmewerkzeug am Ende des Sondenkörpers mit mindestens einem Abstandshalter ausgestattet ist, mit dem das Aufnahmewerkzeug auf den Träger aufsetzbar ist. Der Abstandshalter z. B. in Form eines Vorsprunges, Stifts oder Stegs erfüllt vorteilhafterweise eine Doppelfunktion. Erstens kann die Genauigkeit der Positionierung der Sonde auf dem Träger erhöht werden. Zweitens dient der Abstandshalter als Lauffläche für eine aufzunehmende oder abzulegende Zelle. Der oder die Abstandshalter können so dimensioniert werden, dass jeweils nur eine Zelle zur Aufnahmefläche gelangt. Damit wird die Selektivität des Zelltransfers erhöht.

Gemäß einer weiteren bevorzugten Ausführungsform der Erfindung weist die Aufnahmefläche des Aufnahmewerkzeugs ein Material (Bindungsmaterial) auf, das ein adhärentes Anhaften von Zellen auf der Oberfläche fördert. Das Bindungsmaterial bildet den Sondenkörper, das Aufnahmewerkzeug, mindestens die Vorderseite (Aufnahmefläche) des Aufnahmewerkzeugs oder eine Beschichtung mindestens auf der Vorderseite des Aufnahmewerkzeugs. Es besteht bspw. aus Fibronektin oder Kollagen oder einer Aufrauung mit typischen Dimensionen im nm- bis µm-Bereich. Diese Ausführungsform der Erfindung kann Vorteile in Bezug auf eine Erhöhung der Bindungsgeschwindigkeit beim Zelltransfer besitzen. Das Bindungsmaterial kann alternativ eine Aufrauhung mit charakteristischen Strukturgrößen im Sub-pm-Bereich besitzen, so dass die Anbindung des Zellmaterials an die Sonde gefördert wird. Die Verwendung eines adhäsionsfördernden Materials stellt einen wesentlichen und grundsätzlichen Unterschied des erfindungsgemäßen Werkzeugs gegenüber herkömmlichen Injektionsnadeln dar.

Ein weiterer Vorteil der Erfindung besteht darin, dass die Sonde im Gegensatz zu beispielsweise herkömmlichen Biopsienadeln zur Probenaufnahme nicht auf die Verwendung starrer Glas- Kunststoff- oder Metallmaterialien beschränkt ist. Die Sonde kann vielmehr aus einem zumindest am freien Ende des Sondenkörpers und insbesondere am Aufnahmewerkzeug deformierbaren, flexiblen Material, vorzugsweise aus einem elastisch deformierbaren Material bestehen. Beispielsweise kann das Aufnahmewerkzeug aus einer flexiblen Kunststofflamelle, -folie oder -membran (z. B. Dialysefolie, Polyurethan) bestehen, die für den erfindungsgemäßen Zelltransfer am Träger positioniert wird, bis die mindestens eine Zelle aufgenommen worden ist.

Für die o. g. Untersuchung der Zelle auf oder in der Sonde ist diese vorzugsweise mit mindestens einem Sensor ausgestattet. Der mindestens eine Sensor kann zur Erfassung von chemischen oder physikalischen Eigenschaften von Zellen oder Substanzen im Zellmaterial oder im Inneren des Sondenkörpers eingerichtet sein. Wenn der mindestens eine Sensor, zum Beispiel ein Impedanzsensor im Hohlraum des Sondenkörpers angeordnet ist, können sich Vorteile für die Überwachung einer Injektion oder einer Biopsie ergeben.

Die Sonde kann ferner mindestens einen elektrischen Leiter aufweisen, der bis in die Vorderseite der Sonde verläuft. Der Leiter kann zum Beispiel in die Sonde eingeschmolzen sein und eine Messelektrode bilden. Es kann auch mindestens ein Lichtleiter zum Beispiel für spektroskopische Messungen im Zellmaterial vorgesehen sein.

Erfindungsgemäß kann das Aufnahmewerkzeug mindestens an seiner Vorderseite, die beim Zelltransfer zum Träger gerichtet ist, eine abgerundete Oberfläche besitzen. Gemäß einer bevorzugten Ausführungsform der Erfindung besitzt die Sondenvorderseite in einzelnen Bereichen der abgerundeten Oberfläche einen lokalen Krümmungsradius, der größer als 10 µm ist. Damit ist die abgerundete Oberfläche größer als die Zellarten in den typischerweise behandelten Zellmaterialien, insbesondere Zellarten in Gewebe. Damit wird die Verletzungswahrscheinlichkeit bei der Bewegung der Sonde vermindert. Besondere Vorteile können sich mit einem lokalen Krümmungsradius ergeben, der größer als 20 µm, vorzugsweise größer als 0.1 mm ist. Für die Anwendung bei Zellkulturen ist der Krümmungsradius kleiner als 5 mm, vorzugsweise kleiner als 2 mm.

Wenn gemäß einer weiteren Modifizierung das Aufnahmewerkzeug durchsichtig ist, kann eine optische Beobachtung des Zelltransfers erleichtert werden.

Ein weiterer Gegenstand der Erfindung ist ein Zellmanipulator (insbesondere ein Arbeitsgerät wie zum Beispiel eine Injektions-, Biopsie- und/oder Untersuchungseinrichtung) für die Bearbeitung von Zellmaterial, wobei der Zellmanipulator mindestens eine erfindungsgemäße Sonde, einen Träger zur Aufnahme mindestens einer Zelle oder von Zellmaterial, wie zum Beispiel einer Zellkultur und mindestens eine Antriebseinrichtung zur Einstellung einer Transferanordnung der Sonde oder des Trägers umfasst. Der Zellmanipulator besitzt den besonderen Vorteil, dass die Sonde und der Träger mit der Antriebseinrichtung mit hoher Genauigkeit und Reproduzierbarkeit relativ zueinander positioniert und ggf. bewegt werden können.

Die Antriebseinrichtung ist vorzugsweise zur Bewegung der Sonde und/oder des Trägers mit einer Relativgeschwindigkeit ausgelegt, die kleiner oder gleich der o. g. physiologischen Bezugsgeschwindigkeit der Zelle ist. Dieses Merkmal ist nicht nur von Bedeutung, wenn der Träger und die Zelle relativ zueinander während des Zelltransfers bewegt werden, sondern auch für die Einstellung der Transferposition mit einer relativ zum Träger ruhenden Sonde. Vorteilhafterweise können auch bei der Positionierung der Sonde und des Trägers relativ zueinander Verletzungen am Zellmaterial auf dem Träger vermieden werden.

Wenn die Antriebseinrichtung einen piezoelektrischen Antrieb umfasst, ergeben sich Vorteile für die Genauigkeit und Reproduzierbarkeit der Sondenbewegung. Alternativ kann die Antriebseinrichtung durch einen Magnetantrieb gebildet werden, wodurch sich Vorteile in Bezug auf eine berührungslose Übertragung der Vortriebskraft ergeben können. Des Weiteren kann die Antriebseinrichtung einen Federantrieb, der Vorteile in Bezug auf einen besonders einfachen Aufbau der Sonde bieten kann, oder einen thermischen Antrieb umfassen, bei dem eine Ausdehnung- oder Schrumpfungsbewegung eines Sondenmaterials ausgenutzt wird.

Gemäß bevorzugten Ausführungsformen der Erfindung ist der Zellmanipulator mit einer Positioniereinrichtung zur Ausrichtung der Sonde, des Trägers und der Antriebseinrichtung, und/oder mit einer Detektoreinrichtung zur Erfassung der Position oder Bewegung der Sonde, des Trägers oder von Zellen ausgestattet.

Bevorzugte Anwendungen der Erfindung sind die *in vitro* Zellkultur, das Tissue Engineering in der Biotechnologie, die Schaffung von Gewebemodellen für die Pharmakologie und die medizinische Therapie.

Weitere Einzelheiten und Vorteile der Erfindung werden im Folgenden unter Bezug auf die beigefügten Zeichnungen beschrieben. Es zeigen:
- Figur 1:: eine schematische Illustration einer Kombination aus einer Sonde und einem Träger zur Durchführung eines erfindungsgemäßen Zelltransfers,
- Figur 2:: eine schematische Übersichtsdarstellung eines erfindungsgemäßen Zellmanipulators,
- Figur 3:: den Ablauf von Zelltransferschritten entsprechend einer Ausführungsform des erfindungsgemäßen Verfahrens,
- Figur 4:: ein Flussdiagramm zur Illustration einer Ausführungsform eines erfindungsgemäßen Verfahrens.
- Figur 5:: den Ablauf von Zelltransferschritten entsprechend einer weiteren Ausführungsform des erfindungsgemäßen Verfahrens,
- Figur 6:: weitere Ausführungsformen erfindungsgemäßer Sonden,
- Figur 7:: den Ablauf von Zelltransferschritten bei Verwendung einer erfindungsgemäßen Sonde mit einem ringförmigen Aufnahmewerkzeug,
- Figur 8:: den Ablauf von Zelltransferschritten entsprechend einer weiteren Ausführungsform des erfindungsgemäßen Verfahrens, und
- Figur 9:: eine Illustration von weiteren Einzelheiten einer Ausführungsform eines erfindungsgemäßen Zellmanipulators.

Die Erfindung wird im folgenden unter beispielhaftem Bezug auf Ausführungsformen beschrieben, die die natürliche Zellbewegung vom Träger auf die Sonde und die Funktion des Zellmanipulators illustrieren. Die natürliche Zellbewegung und deren Charakterisierung und Beeinflussung werden nicht erläutert, soweit deren Einzelheiten an sich bekannt sind. Hierzu wird beispielsweise auf die oben zitierte Publikation von L. P. Cramer verwiesen. Es wird betont, dass auch wenn bei den beschriebenen Ausführungsbeispielen meistens auf den Zelltransfer vom Träger auf die Sonde Bezug genommen wird, die Erfindung mit der umgekehrten Bewegungsrichtung von der Sonde auf den Träger entsprechend implementierbar ist.

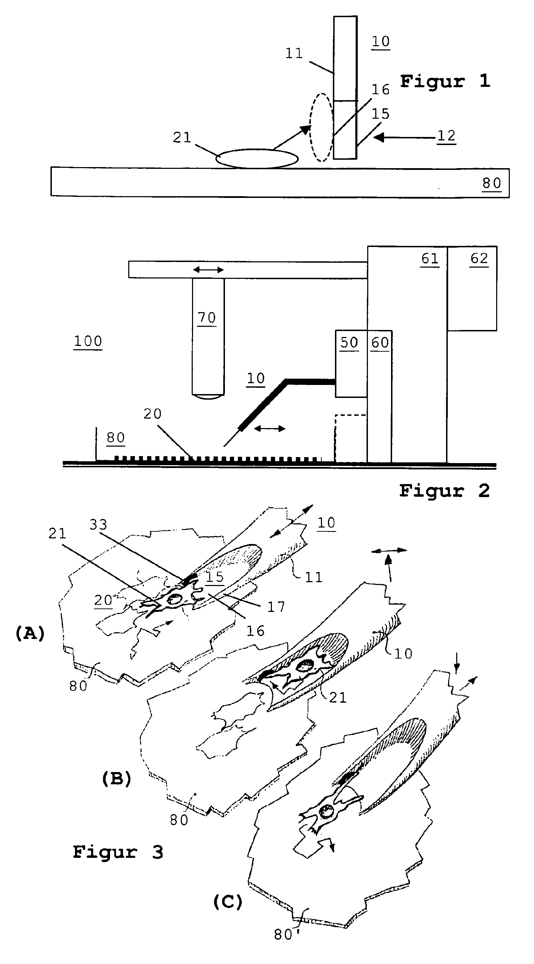

Figur 1 zeigt schematisch als Grundkomponenten zur Ausführung des erfindungsgemäßen Zelltransferverfahrens eine Sonde 10 und einen Träger 80, die so in eine gegenseitige Transferanordnung gebracht werden, dass eine Zelle 21 aufgrund ihrer natürlichen Zellbewegung von der Oberfläche des Trägers 80, z. B. aus einer Zellkultur heraus auf die Sonde 10 wandern kann. Die Sonde 10 umfasst einen Sondenkörper 11, an dessen freien Ende 12 das Aufnahmewerkzeug 15 gebildet ist. Der Träger 80 ist beispielsweise eine Zellkulturschale, auf deren Boden eine Zellkultur gebildet ist oder ein Substrat in einem Kultivierungssystem.

Die Bewegung der Zelle 21 vom Träger 80 auf die Sonde 10 erfolgt derart, dass zunächst Adhäsionskontakte von Zellmembranorganen vom Träger 80 gelöst und auf der Aufnahmefläche 16 des Aufnahmewerkzeugs 15 neu gebildet werden. Im Verlauf der Zellbewegung werden zunehmend Adhäsionskontakte am Träger 80 aufgetrennt, bis sich die Zelle 21 komplett auf dem Aufnahmewerkzeug 15 befindet. Generell ist die natürliche Zellbewegung auf einer isotropen Oberfläche ungerichtet und stochastisch. Zur Umsetzung der Erfindung kann es ausreichend sein, wenn die Zelle im Rahmen der stochastischen Bewegung zufällig auf die Sonde wandert. Aufgrund der hohen Zellbeweglichkeit kann unter praktischen Bedingungen auch in diesem Fall genügend schnell (z. B. innerhalb von 1 bis 2 Stunden) eine Zelle auf die Sonde aufgenommen werden. Bevorzugt werden jedoch die unten erläuterten Maßnahmen zur gerichteten Zellwanderung auf die Sonde oder zur Förderung der Transferbewegung realisiert.

Zur Vermeidung einer unerwünschten Wanderung der Zelle auf dem Sondenkörper kann auf und ggf. in diesem eine Beschichtung oder Behandlung zur Adhäsionsminderung vorgesehen sein. Beispielsweise erfolgt eine Silanisierung oder eine Belegung mit biologischen Makromolekülen (zum Beispiel PolyHema).

Ein erfindungsgemäßer Zellmanipulator 100 umfasst gemäß der schematischen Darstellung in Figur 2 die Sonde 10, die Antriebseinrichtung 50, eine Positioniereinrichtung 60, eine Detektoreinrichtung 70, den Träger 80 mit einer Zellkultur 20 und einer Flüssigkeitsüberdeckung 81 und eine Steuereinrichtung 61. Die Flüssigkeitsüberdeckung 81 umfasst z. B. eine Suspensions- und Kulturflüssigkeit, die vorteilhafterweise den erfindungsgemäßen Zelltransfer nicht behindert.

Die Antriebseinrichtung 50 dient der Einstellung der Transferanordnung von Sonde 10 und Träger 80 und ggf. der Ausübung einer Vortriebskraft an der Sonde 10 während des Zelltransfers. Die Antriebseinrichtung 50 enthält z. B. Piezokristalle, mit denen die Sonde 10 analog zu an sich bekannten Mikromanipulatorsystemen in verschiedenen Raumrichtungen mit einer Genauigkeit von < 100 nm positionierbar und bewegbar sind. Der Arbeitsweg der Sonde 10 kann zwischen 0,1 mm und einigen Zentimetern liegen. Alternativ kann die Antriebseinrichtung 50 (gestrichelt gezeigt) zur Bewegung des Trägers 80 des Zellmaterials eingerichtet sein.

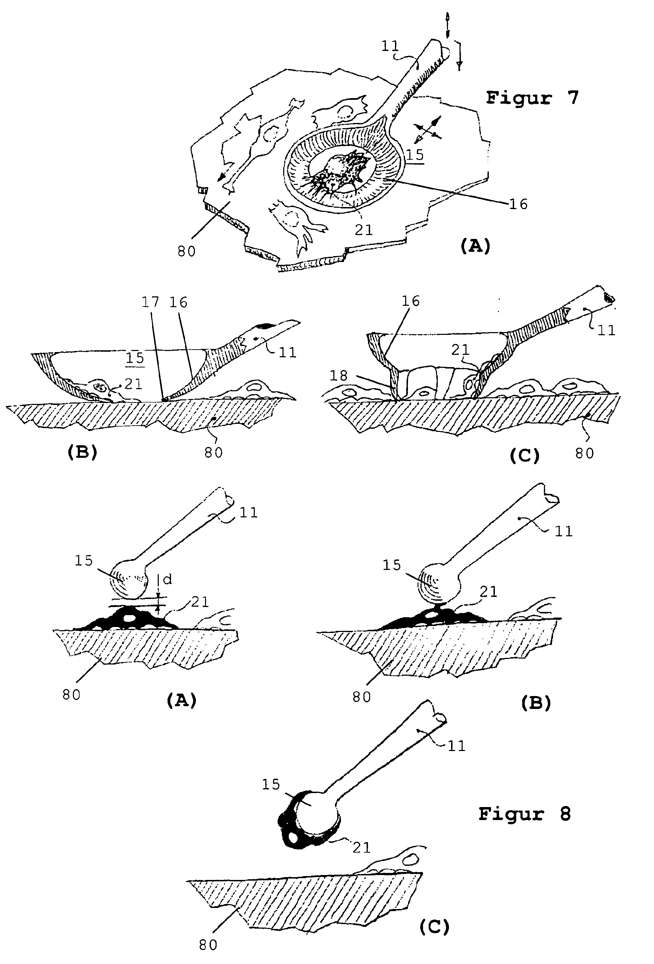

Die Antriebseinrichtung 50 ist mit der Positioniereinrichtung 60 verbunden. Die Positioniereinrichtung 60 ist ein mechanisch stabil und Mikrometer-genau relativ zum Träger 80 fixierbares Bauteil. Seine Aufgabe ist es, die stabile Lage der Sonde relativ zum Träger über die Dauer der Manipulation (Stunden, Tage oder auch Wochen) zu garantieren. Dies kann über eine justierbare Dreipunktlagerung auf dem Kultursystem (siehe Figur 8) oder eine Verankerung z.B. am Knochen des Schädels bei einer Zellinjektion in das Gehirn erreicht werden. Die Positioniereinrichtung 60 ist des Weiteren für Bewegungen der Sonde oder des Trägers über größere Strecken und mit größeren Geschwindigkeiten eingerichtet, die vor oder nach der Einstellung der Transferanordnung von Sonde und Träger gewünscht sind, und enthält hierzu zum Beispiel einen Stellmotor.

Die Positioniereinrichtung 60 ist des Weiteren mit der Steuereinrichtung 61 und einer Mess- und Anzeigeeinrichtung 62 verbunden. Die Steuereinrichtung 61 dient der Steuerung des Gesamtsystems und enthält einen Prozessor oder Computer. Die aktuellen und geplanten Positionen werden über Sensoren (Dehnungsmessstreifen an Aktoren, 4-Quadrantendetektion mittels Laserstrahl wie beim der Atomkraftmikroskopie oder in analoger Weise) erfolgen. Die Information wird Software-basiert verarbeitet und so dargestellt, dass die reale und geplante Bewegung auf einem Monitor zusammen mit zweckdienlichen Parametern dargestellt wird. Hierzu kann als Detektoreinrichtung 70 ein Kamerasystem mit mikroskopischer Vergrößerung und einer Zoom-Funktion vorgesehen sein.

Die Sonde 10 kann mit einem Probenreservoir (nicht dargestellt) verbunden sein, das ein Transportsystem zur Bewegung einer Probe in die Sonde 10 enthält. Die in das Zellmaterial zu transferierende Zelle ist bspw. in einer Zellsuspension enthalten. Das Transportsystem ist eine an sich bekannte Fördereinrichtungen wie z. B. eine Präzisions-Spritzenpumpe. Das Probenreservoir für die Aufnahme der zu injizierenden Zellen kann eine Hamiltonspritze oder ein über ein totvolumenarmes 3-Wegesystem angeschlossenes Behältnis sein. Das Transportsystem drückt beispielsweise über mechanische Kompression eine Zellsuspension in die Injektionswerkzeuge (Sonde 10). Dabei sind sehr geringe Volumina zu bewegen (Geschwindigkeit wenige µm/min). Es können auch Spülvorgänge mit Waschlösungen (Geschwindigkeit 1 bis zu einigen 100 µm/s) ausgeführt werden. Dies kann über programmierbare Spritzenpumpen erreicht werden.

Gemäß Figur 3 ist das Aufnahmewerkzeug 15 der Sonde 10 schaufelförmig am Ende eines kapillarförmigen Sondenkörpers 11 mit einem Hohlkanal 31 gebildet. Das Ende des Sondenkörpers 11 ist so schräg angeschnitten, dass das Aufnahmewerkzeug 15 einen Schieber oder eine Schaufel mit einer konkaven Aufnahmefläche 16 und von dieser abstehenden Rändern 17 bildet. Die Sonde 10 ist mit den Antriebs- und Positioniereinrichtungen 50, 60 (s. Figur 2) in allen Raumrichtungen beweglich und insbesondere entlang der Längsachse des Sondenkörpers 11 verschiebbar (siehe Doppelpfeil).

Der Sondenkörper 11 besteht bspw. aus Glas, einem inerten Metall (z. B. Platin) oder einem inerten Kunststoffmaterial (z. B. Polyimid). Der Innendurchmesser a des Hohlkanals 31 an der Öffnung 32 und der Außendurchmesser b des Sondenkörpers 11 an seinem Ende sind typischerweise in den Bereichen a = 10 µm bis 100 µm und b = 10.5 µm bis 200 µm gewählt. Es können kleinere Maße im Bereich a = 0.1 µm bis 10 µm für den Transfer von besonders kleinen Zellen oder von synthetischen Partikeln oder größere Maße von z. B. a = 100 µm bis 5 mm für den Transfer z. B. von embryonalen Stammzellen gewählt werden, wobei das Maß b entsprechend der Wandstärke des Sondenkörpers 11 entsprechend größer gewählt ist.

Die Sonde 10 kann mit einem Impedanzsensor 33 ausgestattet sein. Der Impedanzsensor 33 umfasst zwei halbkreisförmige Elektroden (Impedanzelektroden), die auf der Oberfläche der Vorderseite des Werkzeugs 15 bspw. durch Aufdampfen angebracht sind. Alternativ können die Impedanzelektroden auf der Außenseite des Sondenkörpers 11 an dessen Ende angebracht sein. Über elektrische Verbindungsleitungen (nicht gezeigt) entlang dem Sondenkörper 11 sind die Elektroden des Impedanzsensors 33 mit der Steuereinrichtung verbunden. Beim Durchwandern einer Zelle 21 durch den Hohlkanal 31 wird durch die Verstimmung der Impedanz zwischen den Impedanzelektroden ein Impedanzsignal generiert, das in an sich bekannter Weise Auskunft über die Größe und die elektrischen Eigenschaften der vorbeitretenden Zelle gibt.

Die Aufnahmefläche 16 trägt eine adhäsionsfördernde Beschichtung. Diese Verbindungsbeschichtung ist vorzugsweise nur auf der Aufnahmefläche 16 vorgesehen, während die Oberfläche des übrigen Sondenkörpers 11 vorzugsweise adhäsionsmindernd beschichtet ist, um ein Aufwandern zu minimieren und ggf. ein Gleiten zwischen verdrängten Zellen zu fördern. Die Verbindungsbeschichtung besteht zum Beispiel aus Fibronektin. Zur Adhäsionsminderung kann eine Silanisierung oder eine Belegung mit biologischen Makromolekülen (zum Beispiel PolyHema, PTFE) vorgesehen sein. Auch die innere Oberfläche des Hohlkanals kann in dieser Weise adhäsionsmindernd beschichtet sein. An der Vorderkante (siehe Teilbild B) besitzt die Aufnahmefläche 16 eine Krümmung. Durch die entsprechende Verlängerung der Vorderkante wird das Aufwandern von Zellen vorteilhafterweise begünstigt.

Die Querschnittsform der kapillarförmigen Sondenkörper ist vorzugsweise rund. Alternativ kann eine abgeflachte Ellipsenform vorgesehen sein, die eine Vorzugsrichtung zur seitlichen Bewegung in einer Bewegungsrichtung senkrecht zur Längsachse des Sondenkörpers 11 ermöglicht.

Figur 3 zeigt in den drei Teilbildern A - C die einzelnen Phasen eines Zelltransfers von einem Träger 80 mit einer Sonde 10 auf ein Zielsubstrat 80' (jeweils ausschnittsweise dargestellt). Auf dem Träger 80 befindet sich eine Zellkultur 20 mit mehreren Zellen, von denen eine Zelle 21 auf das Zielsubstrat 80' übertragen werden soll. Auf dem Träger kann eine Flüssigkeitsüberdeckung vorgesehen sein (siehe Figur 2). Die Zelle 21 ist beispielsweise eine im Zellmaterial 20 aufgefundene Stammzelle.

Die Zelle 21 führt zunächst eine stochastische Zellbewegung auf dem Substrat 80 aus, bis sie auf das Aufnahmewerkzeug 15 trifft und aufgrund der eigenen Zellbewegung auf dieses wandert. Dieser Zelltransfer kann durch die adhäsionsfördernde Beschichtung (siehe oben) auf der Aufnahmefläche 16 und/oder eine zur Zellbewegung gegenläufige Vorschubbewegung der Sonde 10 gefördert werden. Über die natürlichen Fluktuationen der Adhäsionskontakte der Zelle 21 werden diese allmählich hin zum Aufnahmewerkzeug 15 umgeordnet. Sobald die Zelle 21 vollständig auf der Sonde angeordnet ist (Teilbild B), kann die Sonde 10 nunmehr mit erhöhter Geschwindigkeit vom Träger 80 zurückgezogen werden. Der weitere Transport erfolgt in Abhängigkeit von der konkreten Anwendung zum Zielsubstrat 80', wo die Zelle 21 aufgrund ihrer stochastischen Bewegung die Sonde wieder verlässt.

Die Bewegung der Zelle 21 von der Sonde 10 auf das Zielsubstrat 80' wird vorteilhafterweise bereits durch die natürliche Aggregationstendenz von Zellen gefördert, falls auf dem Zielsubstrat 80' bereits mindestens eine Zelle vorhanden ist. Alternativ kann auf dem Zielsubstrat 80' ein molekularer Attraktor, wie zum Beispiel Signalmoleküle, Nährstoffe, Hormone oder chemotaktisch wirkende Substanzen angeordnet sein, um den gerichteten Transfer zu fördern.

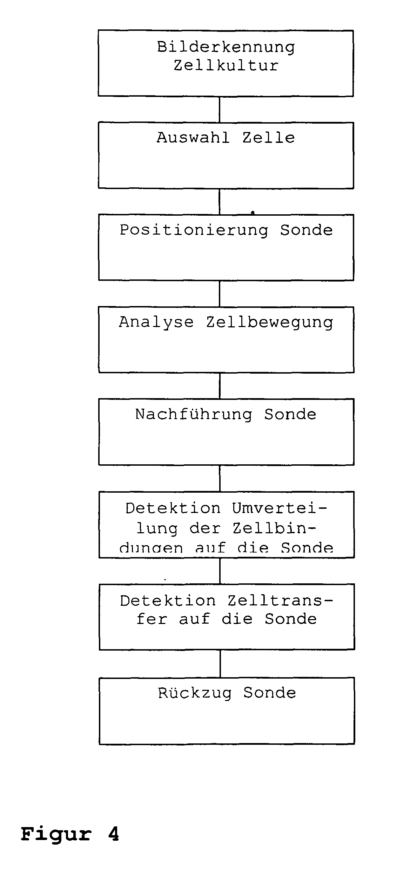

Ein Verfahrensablauf des Zelltransfers ist mit weiteren Einzelheiten in Figur 4 beispielhaft angegeben. Nach einer Bilderkennung an der Zellkultur 20 wird eine gewünschte Zelle 21 (z. B. eine Fibroblasten- oder Stammzelle) ausgewählt. Es folgen eine Positionierung der Sonde 10 in der Nähe der ausgewählten Zelle 21 und eine optische Messung und Analyse der Zellbewegung auf dem Träger 80, um die Sonde 10 relativ zur Zellbewegung fein zu justieren und nachzuführen. Sobald erfasst wurde, dass Zellkontakte auf die Sonde umgeordnet wurden, wird die Richtung der Sondennachführbewegung so eingestellt, dass der Transfer der Zelle begünstigt wird. Nachdem erfasst wurde, dass die Zelle komplett auf die Sonde 10 übertragen wurde erfolgt ein Rückzug der Sonde 10 vom Träger 80. Die Bilderkennungs- und Detektionsschritte erfolgen unter Verwendung der Detektoreinrichtung 70 (siehe Figur 2).

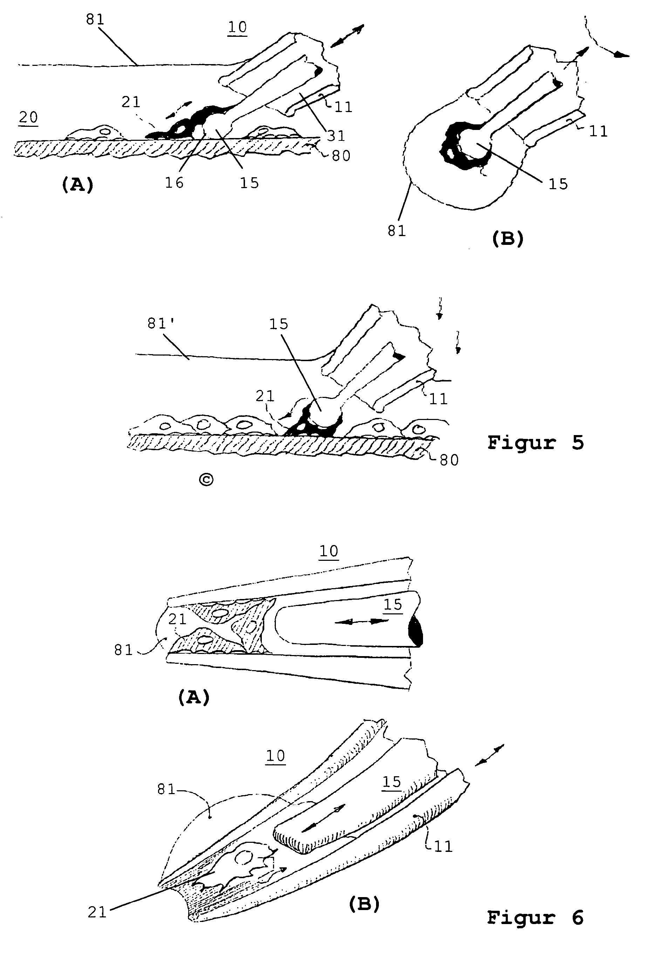

Figur 5 illustriert in Teilbilder A - C den Zelltransfer mit einer abgewandelten Sonde 10. Im Hohlkanal 31 des kapillarförmigen Sondenkörpers 11 befindet sich das kugelförmige Aufnahmewerkzeug 15, dessen Oberfläche die Aufnahmefläche 16 bildet. Das Aufnahmewerkzeug 15 besitzt einen Durchmesser von zum Beispiel 15 µm. Das Aufnahmewerkzeug 15 ist im kapillarförmigen Sondenkörper 11 mit der Antriebseinrichtung (siehe Figur 2) verschiebbar.

Gemäß Teilbild A wird die Sonde 10 in die Kultivierungsflüssigkeit 81 eingetaucht und zum Substrat 80 mit der Zellkultur 20 bewegt, bis sich das vordere Ende der Sonde 10 mit einem Abstand oberhalb der Oberfläche der Zellkultur 20 befindet. Anschließend wird das Aufnahmewerkzeug 15 aus dem freien Ende des Hohlkanals 31 herausbewegt, bis es auf der Oberfläche des Trägers 80 aufsitzt. Bei dieser Vortriebsbewegung erfolgt eine verletzungsfreie Verdrängung von ggf. entlang des Weges des Aufnahmewerkzeugs 15 befindlichen Zellmaterial. Die Zelle 21 wandert aufgrund ihrer natürlichen Eigenbewegung auf die Aufnahmefläche 16. Wenn sich die Zelle 21 vollständig auf dem Aufnahmewerkzeug 15 befindet, kann die Sonde 10 vom Träger 80 abgehoben werden. Die Rückzugsbewegung erfolgt wiederum verletzungsfrei für die zurückbleibenden Zellen der Zellkultur 20. Im abgehobenen Zustand bildet die Kultivierungsflüssigkeit 81 vorteilhafterweise einen Flüssigkeitstropfen um das Aufnahmewerkzeug 15 (Teilbild B). Die Zelle ist dadurch geschützt. Das Aufnahmewerkzeug 15 kann während der Bewegung zum Zielsubstrat 80' in der vorgeschobenen Position bleiben oder zurückgezogen werden. Am Zielsubstrat 80' erfolgt ein Eintauchen der Sonde 10 in die Suspensionsflüssigkeit 81' (Teilbild C). Analog zum oben beschriebenen Vorgang kann nach ausreichender Annäherung des Aufnahmewerkzeugs 15 an das Zielsubstrat 80' die Zelle 21 übertragen werden.

Analog zu den in Figur 3 und 5 gezeigten Vorgängen können Zellen auf oder in ein Gewebe eingebracht werden.

Figur 6 illustriert ein Zusammenwirkung des Sondenkörpers 11 mit dem Aufnahmewerkzeug 15 am Beispiel von zwei abgewandelten Ausführungsformen erfindungsgemäß verwendeter Sonden 10. Gemäß Teilbild A ist der Sondenkörper 11 eine Kapillare, deren Ende im Schnitt gezeigt ist. Der Innenraum (Hohlkanal 31) der Kapillare ist flüssigkeitsgefüllt. Das Aufnahmewerkzeug 15 ist ein in der Kapillare beweglicher Stößel. In Teilbild B ist das entsprechende Prinzip mit einem offenen Schieber gezeigt, der beispielsweise analog zu Figur 3 am Ende einer Kapillare gebildet ist und in dem ebenfalls ein Stößel beweglich ist. In beiden Fällen ist die mindestens eine Zelle 21 auf der Sonde mit einem Flüssigkeitstropfen 81 bedeckt.

Beim erfindungsgemäßen Zelltransfer kann der Stößel 15 analog zu Figur 6 vorgeschoben und zurückgezogen werden, um Zellen von einer Kultur aufzunehmen. Alternativ kann die erfindungsgemäß vorgesehene aktive Zellwanderung zuerst in den Hohlkanal 31 und in diesem auf den Stößel 15 erfolgen. Bei der Ablage auf einem Zielsubstrat kann der Stößel 15 verwendet werden, um Zellen mittels verletzungsfreier Verdrängung aus der Kapillare 11 herauszuschieben.

Eine wichtige Anwendung der Erfindung besteht bei der verletzungsfreien Entnahme von Zellen aus Gewebe (verwundungsfreie Biopsie). Ein besonderer Vorteil der Erfindung besteht darin, dass selbst größere Werkzeuge mit Dimensionen, die größer als die aufzunehmenden Zellen sind, zur Entnahme von Zellmaterial z. B. für eine Diagnose verwendet werden können. Figur 7 illustriert beispielhaft zwei Varianten eines erfindungsgemäß verwendeten Aufnahmewerkzeugs mit einer ringförmigen Aufnahmefläche 16.

Gemäß den Teilbildern A und B der Figur 7 besitzt das Aufnahmewerkzeug 15 eine Querschnittsform entsprechend dem Ausschnitt einer Kugelfläche. Die Aufnahmefläche 16 ist auf der Innenseite der Kugelfläche gebildet. Der innere Rand 17 der Aufnahmefläche 16 bildet eine Öffnung, mit der die Sonde 10 zum erfindungsgemäßen Zelltransfer auf den Träger 80 aufgesetzt wird. Gemäß Teilbild A wird das Aufnahmewerkzeug 15 mit der physiologischen Bezugsgeschwindigkeit langsam über einer gewünschten Zelle 21 abgesetzt. Diese wird sich dann aufgrund ihrer natürlichen Zellbewegung vom Träger 80 auf die Aufnahmefläche 16 bewegen. Sobald dieser Transfer abgeschlossen ist (Teilbild B), kann die Sonde 10 vom Träger 80 entfernt werden.

Teilbild C der Figur 7 illustriert die Verwendung von Abstandshaltern 18, mit denen das Aufnahmewerkzeug 15 auf die Oberfläche des Trägers 80 aufsetzbar ist. Die Abstandshalter 18 erleichtern das Aufsetzen des Aufnahmewerkzeugs 15 und das selektive Aufwandern einzelner Zellen 21.

Das Aufnahmewerkzeug 15 besteht aus einem der o. g. Materialien, es kann einen Durchmesser im Bereich von 10 µm bis 10 mm und eine Höhe von 0.5 mm bis 10 mm besitzen.

Figur 8 illustriert, dass das Aufnahmewerkzeug 15 für den erfindungsgemäßen Zelltransfer nicht notwendigerweise auf eine Zellkultur aufgesetzt werden muss. Vielmehr ist es möglich, gemäß Teilbild A die Transferanordnung zuerst mit einem Abstand d zwischen dem Aufnahmewerkzeug 15 und der Zelle 21 einzustellen. Der Abstand d kann beispielsweise im Bereich von einigen hundert nm bis einige Mikrometer (z. B. 5 µm) gewählt werden. Anschließend erfolgt gemäß Teilbild B eine kurzzeitige Berührung (Dauer 5 s bis einige Minuten) der Aufnahmefläche 16 mit der Zelle 21, um den Zelltransfer zu starten. Die Erfinder haben festgestellt, dass ein adhärentes Anheften an jedem Ort der Zelloberfläche möglich ist und dass der Zelltransfer über den Abstand d erfolgen kann. Beim illustrierten Beispiel ist das Aufnahmewerkzeug 15 vorzugsweise aus einem transparenten Material gebildet, um den Vorgang des Anheftens und des Zelltransfers optisch, z. B. mit einem Mikroskop beobachten zu können. Für eine verzerrungsfreie Beobachtung besitzt das Aufnahmewerkzeug 15 eine planare Oberfläche, es wird zum Beispiel durch eine plane Platte gebildet.

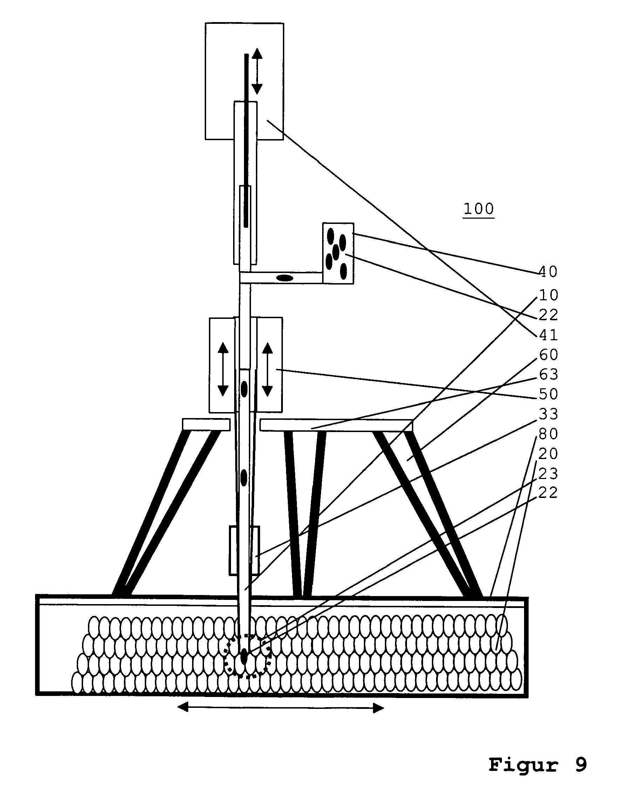

In Figur 9 sind weitere Einzelheiten des Zellmanipulators 100 am Beispiel eines *in vitro* Systems für die Biotechnologie (Tissue Engineering) gezeigt. Ziel ist es, in einen Zellgewebeverband 20 mittels einer Mikrokapillare (Sonde 10) eine oder mehrere Zellen 22 aus einem Zellreservoir 40 zu injizieren. Die Sonde 10 ist über ein Kanalsystem mit dem Zellreservoir 40 verbunden. Gleichzeitig ist die Sonde 10 an eine Hamiltonspritze (Transportsystem 41 für die Zellsuspension 22) angeschlossen, die über einen Prozessor oder eine Computersteuerung angesprochen wird (nicht dargestellt). Das gesamte System befindet sich auf einer Arbeitsplattform 63, die sich nicht relativ zum Gewebeverband 20 bewegen kann, da sie über die Positioniereinrichtung 60 fest mit dem Träger (Kulturschale 80) verbunden ist.

Die Grundjustage der Arbeitsplattform 63 erfolgt mit der Positioniereinrichtung 60. Als Vortriebssystem oder Antriebseinrichtung 50 für die Sonde 10 dient ein Piezorohr, das sich in der angegebenen Weise ausdehnen oder verkürzen kann und dadurch die Sonde 10 in den Gewebeverband 20 einführt bzw. herauszieht. Dargestellt ist ein Zielzellgebiet 23 (gepunkteter Ring), in den gerade eine Zelle 22 eingefügt wird. Am Schaft der Kapillar-Sonde 10 befindet sich ein Einzelzelldetektorsystem 33, mit dem die Zahl der injizierten Zellen erfasst werden kann (hier als optisches System dargestellt).

Der Zellmanipulator 100 entsprechend Figur 9 wird wie folgt betätigt. In einem ersten Schritt wird die Positioniereinrichtung 60 mit dem Zielgewebe 20 in eine feste Verbindung gebracht. Dies erfolgt durch Fixieren der Positioniereinheit an der Kulturschale 80 oder an der Oberfläche eines Organismus bzw. einem geeigneten Teil des Knochengerüstes. In einem zweiten Schritt wird das Mikroinjektionswerkzeug 10 an der Arbeitsplattform 61 befestigt und grob vorjustiert, so dass es kurz vor dem Zielgewebe- oder -zellsystem 20 mit seiner Spitze lokalisiert ist. Im nächsten Schritt wird die Kapillare der Sonde 10 mit der Nährlösung, einer physiologischen Lösung oder einem anderen geeigneten Fluid befüllt und an das Zielgewebe bis zu dessen Berührung herangeführt. Danach erfolgt der programmierte Vortrieb des Mikroinjektionswerkzeuges entsprechend der Geschwindigkeit von zum Beispiel weniger als 1 µm/h bis zu einigen 100 µm/h, bis der Zielbereich 23 erreicht ist. Entweder zu diesem Zeitpunkt oder früher werden die zu injizierenden Zellen aus dem Reservoir in das Mikroinjektionswerkzeug eingespült und über den Detektor beim Passieren des eingestochenen Teils erfasst, gezählt und ggf. charakterisiert. Durch Bewegen des Injektionswerkzeuges in alle drei Raumrichtungen mit der o. g. geringen Geschwindigkeit kann das System in ein neues Zielgebiet geführt werden, so dass in einer vorher bestimmbaren Weise Zellen dreidimensional im Gewebe positioniert werden. Am gewünschten Ort im Zielgewebe erfolgt ein Transfer von mindestens einer Zelle durch deren natürliche Eigenbewegung, ggf. unterstützt durch einen von der Suspensionsflüssigkeit ausgeübten Druck. Nach erfolgter Injektion wird das Mikroinjektionswerkzeug wieder aus dem Zielgewebe entfernt. Die geringe Geschwindigkeit, die auch als "physiologische Geschwindigkeit" bezeichnet wird, erlaubt den Zellen des Gewebes an der Spitze des Injektionswerkzeuges das Lösen der Zelladhäsionskontakte, so dass es zu einer geordneten Verdrängung der Zellen kommt, nicht aber deren Verletzung. Die in der vorstehenden Beschreibung, den Ansprüchen und den Zeichnungen offenbarten Merkmale der Erfindung können sowohl einzeln oder auch in Kombination für die Verwirklichung der Erfindung in ihren verschiedenen Ausgestaltungen von Bedeutung sein.

## Patentansprüche

1. Zelltransferverfahren, bei dem ein Transfer von mindestens einer, eine natürliche Zellbewegung ausführenden biologischen Zelle (21) zwischen einem Träger (80) und einer Sonde (10) vorgesehen ist,
**dadurch gekennzeichnet, dass**
- eine Positionierung der Sonde (10) und des Trägers (80) relativ zueinander in eine Transferanordnung vorgesehen ist,
- der Transfer der Zelle (21) von dem Träger (80) auf die Sonde (10) oder umgekehrt aufgrund von natürlichen Zellbewegungen der Zelle erfolgt, wobei
- der Transfer der Zelle (21) außerhalb eines tierischen oder menschlichen Organismus durchgeführt wird.

2. Sonde (10) zum Transfer von mindestens einer, eine natürliche Zellbewegung ausführenden biologischen Zelle (21) von oder zu einem Träger (80), die einen relativ zum Träger (80) beweglichen Sondenkörper (11) aufweist,
**dadurch gekennzeichnet, dass**
der Sondenkörper (11) an einem freien Ende (12) ein Aufnahmewerkzeug (13) mit einer Aufnahmefläche zum adhärenten Anhaften von mindestens einer biologischen Zelle (21) aufweist.

3. Sonde nach Anspruch 2, bei welcher der Sondenkörper (11) einen Stab mit einer langgestreckten Form, an dessen freien Ende das Aufnahmewerkzeug (13) angeordnet ist, oder einen Hohlkörper mit einer langgestreckten Form umfasst.

4. Sonde nach einem der Ansprüche 2 oder 3, bei der das Aufnahmewerkzeug (13) als Teil des Sondenkörpers (11) an dessen freien Ende gebildet ist.

5. Sonde nach Anspruch 4, bei der das Aufnahmewerkzeug (13) im Hohlkörper beweglich angeordnet ist.

6. Sonde nach mindestens einem der Ansprüche 2 bis 5, bei der das Aufnahmewerkzeug (13) eine konkave oder eine konvexe Aufnahmefläche (16) aufweist.

7. Sonde nach mindestens einem der Ansprüche 2 bis 6, bei der das Aufnahmewerkzeug (13) ringförmig gebildet ist.

8. Sonde nach mindestens einem der Ansprüche 2 oder 7, bei der das Aufnahmewerkzeug Abstandshalter (18) zum Aufsetzen auf den Träger (80) aufweist.

9. Sonde nach mindestens einem der Ansprüche 2 bis 8, die aus einem deformierbaren Material besteht.

10. Sonde nach mindestens einem der Ansprüche 2 bis 9, die an einer Vorderseite (13) eine Beschichtung (14) trägt, die ein Anhaften von Zellen fördert.

11. Sonde nach mindestens einem der Ansprüche 2 bis 10, die mindestens einen Sensor (33) aufweist.

12. Sonde nach mindestens einem der Ansprüche 2 bis 11, bei der das Aufnahmewerkzeug (15) mindestens an einer Vorderseite (15), die beim Transfer zum Träger (80) gerichtet ist, eine abgerundete Oberfläche besitzt, insbesondere eine abgerundete Oberfläche mit einem Krümmungsradius, der größer als 10 µm ist.

13. Sonde nach mindestens einem der Ansprüche 2 bis 12, bei der das Aufnahmewerkzeug (15) aus einem transparenten Material besteht.

14. Zellmanipulator zum Transfer von mindestens einer, eine natürliche Zellbewegung ausführenden biologischen Zelle (21) zwischen einem Träger (80) und einer Sonde, mit:
- einer Sonde (10) nach mindestens einem der Ansprüche 2 bis 13,
- einem Träger (80) zur Aufnahme von Zellmaterial,
- einer Antriebseinrichtung (50) zur Positionierung der Sonde (10) und des Trägers (80) in einer Transferanordnung, bei welcher der Transfer der Zelle (21) aufgrund der natürlichen Zellbewegung erfolgt, wobei die Antriebseinrichtung (50), die beispielsweise einen piezoelektrischen Antrieb oder einen Magnetantrieb umfasst, zur Bewegung der Sonde (10) und/oder des Trägers (80) mit einer Relativgeschwindigkeit, die kleiner oder gleich einer physiologischen Bezugsgeschwindigkeit der Zellbewegung ist, insbesondere einer Relativgeschwindigkeit im Bereich von 0.1 µm/h bis 1 mm/h, eingerichtet ist, und optional
- einer Detektoreinrichtung (70) zur Erfassung der Position oder Bewegung der Zelle auf der Sonde (10) oder dem Träger (80), wobei die Antriebseinrichtung (50) und die Detektoreinrichtung (70) vorzugsweise mit einer Steuereinrichtung (61) verbunden sind und die Antriebseinrichtung (50) in Abhängigkeit von Signalen der Detektoreinrichtung (70) betätigbar ist.

15. Zellmanipulator nach Anspruch 14, bei dem die Antriebseinrichtung (50) dazu eingerichtet ist, die Sonde (10) und den Träger (80) relativ zueinander so zu positionieren, dass der Träger die Sonde (10) berührt oder dass der Träger einen Abstand von der Sonde (10) besitzt, der kleiner oder gleich der Ausdehnung der mindestens einen Zelle (21) über dem Träger ist.
